# EUROPÄISCHE PATENTSCHRIFT

(11) **EP 3 738 580 B1**
(45) Veröffentlichungstag und Bekanntmachung des Hinweises auf die Patenterteilung: **14.01.2026**
(21) Anmeldenummer: 20174443.0
(22) Anmeldetag: 13.05.2020
(51) Int. Cl.: A61K 9/00, A61K 31/506, A61K 39/395, A61K 47/36, A61P 27/06, A61P 27/02, A61K 31/404, A61K 31/44, A61K 31/502

(54) **OPHTHALMOLOGISCHES IMPLANTAT ZUR VERWENDUNG BEI DER BEHANDLUNG EINER AUGENKRANKHEIT UND IMPLANTATIONSWERKZEUG MIT EINEM SOLCHEN OPHTHALMOLOGISCHEN IMPLANTAT**
OPHTHALMOLOGICAL IMPLANT FOR USE IN THE TREATMENT OF AN OCULAR DISEASE AND IMPLANTATION TOOL COMPRISING SUCH AN OPHTHALMOLOGIC IMPLANT
IMPLANT OPHTALMOLOGIQUE DESTINÉ À ÊTRE UTILISÉ DANS LE TRAITEMENT D'UNE MALADIE OCULAIRE ET OUTIL D'IMPLANTATION DOTÉ D'UN TEL IMPLANT OPHTALMOLOGIQUE

(30) Priorität: 16.05.2019 DE 102019112917
(43) Veröffentlichungstag der Anmeldung: 18.11.2020
(73) Patentinhaber: Carl Zeiss Meditec AG, 07745 Jena (DE)
(72) Erfinder: WOLFSTEIN, André, 13507 Berlin (DE)
(74) Vertreter: Hofstetter, Schurack & Partner

(56) Entgegenhaltungen:
- EP-A1- 2 446 890
- CA-A1- 3 071 648
- US-A1- 2014 322 206
- US-B2- 8 039 010

## Beschreibung

### Technisches Gebiet

Die Erfindung betrifft ein ophthalmologisches Implantat zur Verwendung bei der Behandlung einer Augenkrankheit, welche Angiogenese umfasst, insbesondere zur Behandlung der feuchten Makuladegeneration. Die Erfindung betrifft weiterhin ein Implantationswerkzeug mit einem solchen ophthalmologischen Implantat.

### Stand der Technik

Angiogenese beschreibt in Abgrenzung zur Vaskulogenese die Bildung neuer Blutgefäße aus vorbestehenden Blutgefäßen und ist Bestandteil sowohl physiologischer als auch pathologischer Prozesse. Die Gefäßneubildung wird generell stimuliert durch wachstumsfördernde Substanzen, die eine Endothelproliferation und Endothelmigration bewirken. Unter dem Begriff Makuladegeneration wird eine Gruppe von Erkrankungen der Netzhaut des Auges zusammengefasst, die die Makula (Macula lutea, "Gelber Fleck") betreffen. Unterschieden werden dabei die trockene und die feuchte Makuladegeneration. Temporal von der Papille gelegen enthält die Makula den "Punkt des schärfsten Sehens" (Fovea centralis) der Netzhaut. Diese enthält die auf die Wahrnehmung von farbigem Licht spezialisierten Sinneszellen (Zapfen), deren Funktionsverlust zum Nachlassen der zentralen Sehschärfe und in vielen Fällen zu Sehbehinderung und sogar Blindheit führt. Die trockene Makuladegeneration ist durch eine geographische Atrophie des retinalen Pigmentepithels gekennzeichnet. Bei der feuchten Makuladegeneration tritt dagegen eine Ödembildung unter der Netzhaut auf. Dabei wachsen Blutgefäße aus der Choroidea in die Netzhaut ein, die man auch als choroidale Neovaskularisationen bezeichnet. Diese Neovaskularisationen führen zur Abhebung der Netzhaut und des Pigmentepithels.

Therapieverfahren zur Behandlung der feuchten Makuladegeneration und anderer Augenkrankheiten, bei denen Angiogenese eine Rolle spielt, befinden sich derzeit in der klinischen Prüfung. Dabei werden Angiogenesehemmer mehrfach während einer bestimmten Zeitdauer in den Glaskörperraum injiziert.

Aus den Dokumenten US 8 039 010 B2, CA 3 071 648 A1, US 2014/322206 A1 und EP 2 446 890 A1 sind verschiedene Implantate und Behandlungsarten für Augenerkrankungen bekannt.

Neben einer hohen Unannehmlichkeit für den Patienten besteht durch eine solche Behandlung ein vergleichsweise hohes Endophthalmitis-Risiko. Zudem weist keine der etablierten Therapieoptionen zufriedenstellende Ergebnisse im Sinne einer Heilung auf. Die betroffenen Patienten sind daher meistens trotzdem auf vergrößernde Sehhilfen (Lupenbrille o.ä.) angewiesen, um mit dem erhaltenen Sehvermögen auszukommen.

### Darstellung der Erfindung

Aufgabe der vorliegenden Erfindung ist es, eine verbesserte Behandlung von Augenkrankheiten, welche Angiogenese umfassen, zu ermöglichen.

Die Aufgabe wird erfindungsgemäß durch ein ophthalmologisches Implantat mit den Merkmalen des Patentanspruchs 1 sowie durch ein Implantationswerkzeug mit den Merkmalen des Patentanspruchs 11 gelöst. Vorteilhafte Ausgestaltungen mit zweckmäßigen Ausbildungen der Erfindung sind in den jeweiligen Unteransprüchen angegeben, wobei vorteilhafte Ausgestaltungen jedes Erfindungsaspekts als vorteilhafte Ausgestaltungen des jeweils anderen Erfindungsaspekts anzusehen sind.

Ein erster Aspekt der Erfindung betrifft ein ophthalmologisches Implantat gemäß Anspruch 1 zur Verwendung bei der Behandlung einer Augenkrankheit, welche Angiogenese umfasst, insbesondere zur Behandlung der feuchten Makuladegeneration. Das erfindungsgemäße ophthalmologische Implantat weist einen im Bereich einer Retina eines menschlichen oder tierischen Patienten anzuordnenden Grundkörper, der zumindest teilweise aus wenigstens einem hydrophilen Polymer besteht, und einen am Grundkörper angeordneten Angiogenesehemmer auf. Ein derartiges ophthalmologisches Implantat ermöglicht eine erhöhte Verweilzeit des Angiogenesehemmers an einer Stelle, an der eine Hemmung der Neovaskularisierung erwünscht und notwendig ist. So verteilt beispielsweise eine herkömmliche Wirkstoffinjektion in den Glaskörper den Wirkstoff im gesamten Glaskörperraum, obwohl dieser beispielsweise nur im Bereich der Makula benötigt wird, das heißt am Ort des unerwünschten Gefäßwachstums. Das erfindungsgemäße ophthalmologische Implantat, das auch als Retina-Patch oder Retina-Pflaster bezeichnet werden kann, kann nicht nur als Versiegelungsmittel für Netzhautrisse fungieren, sondern auch den Angiogenesehemmer in vergleichsweise hoher Konzentration in die unmittelbare Nähe der Makula bringen und dort über einen längeren Zeitraum halten. Dadurch kann die Menge des Angiogenesehemmers auf eine therapeutisch notwendige Menge reduziert werden, wodurch trotz einer verbesserten und langanhaltender Wirksamkeit erhebliche Kosteneinsparungen realisierbar sind. Unter einem hydrophilen Polymer sind dabei Polymere zu verstehen, die unter Normalbedingungen (STP, DIN 1343) gegenüber Wasser einen Kontaktwinkel kleiner 90° besitzen. Dementsprechend wird im Folgenden unter einem hydrophoben Polymer ein Polymer verstanden, das unter Normalbedingungen (STP, DIN 1343) gegenüber Wasser einen Kontaktwinkel größer 90° besitzt. Unter einem Angiogenesehemmer werden vorliegend Arznei- und Wirkstoffe verstanden, die die Bildung neuer Blutgefäße (Angiogenese) unterdrücken können. Generell sind "ein/eine" im Rahmen dieser Offenbarung als unbestimmte Artikel zu lesen, also ohne ausdrücklich gegenteilige Angabe immer auch als "mindestens ein/mindestens eine". Umgekehrt können "ein/eine" auch als "nur ein/nur eine" verstanden werden. Der Begriff "umfassen" ist im Rahmen der vorliegenden Offenbarung generell so auszulegen, dass die entsprechenden Merkmale enthalten sind, das Vorhandensein anderer Merkmale aber nicht ausgeschlossen ist. Umgekehrt kann der Begriff "umfassen" im Rahmen der vorliegenden Offenbarung aber auch im Sinne von "bestehend aus" bzw. von "bestehend im Wesentlichen aus" ausgelegt werden, dass heißt dass neben den im Anschluss an diese Formulierung genannten Merkmalen keine weiteren Merkmale ("bestehen aus") vorhanden sein können oder dass bestimmte weitere Merkmale vorhanden sein können, nämlich solche, die die wesentlichen Merkmale des Erfindungsgegenstands nicht signifikant verändern ("bestehend im Wesentliche aus"). Eine verbesserte mechanische Stabilität und damit eine sicherere Handhabung des Implantats wird erfindungsgemäß dadurch erreicht, dass wenigstens eine Trägerfolie auf zumindest einem Teil einer Oberfläche des Grundkörpers aufpolymerisiert ist, um den Grundkörper zu stabilisieren, wobei die wenigstens eine Trägerfolie auf einer von der Retina abzuwendenden Seite des Grundkörpers auf diesen aufgebracht ist. Mit anderen Worten ist die Trägerfolie im implantierten Zustand des Implantats auf der von der Retina abgewandten Seite des Grundkörpers angeordnet, wodurch die Abgabe und/oder Wechselwirkung des Angiogenesehemmers mit der Retina nicht durch die Trägerfolie beeinträchtigt wird. Vorzugsweise ist die Trägerfolie biologisch abbaubar. Die Trägerfolie kann in manchen Ausführungen eine Dicke zwischen etwa 0,01 mm und etwa 0,5 mm, beispielsweise 0,05 mm besitzen. Erfindungsgemäß ist weiterhin vorgesehen, dass der wenigstens eine Angiogenesehemmer ausgewählt ist aus einer Gruppe, die Bevacizumab, Brolucizumab, Ranibizumab, Ramucirumab, Aflibercept, Pegaptanib, Thalidomid, Axitinib, Lenvatinib, Lucitanib, Motesanib, Pazopanib, Regorafenib, Sorafenib, Sunitinib, Tivozanib, Vatalanib und Biosimilars davon umfasst. Hierdurch können insbesondere Tyrosinkinasen gehemmt werden, wobei manche der genannten Verbindungen als Multikinase-Inhibitoren mehrere Proteinkinasen verschiedener Klassen hemmen können und dadurch eine verbesserte Wirksamkeit aufweisen können.

In einer vorteilhaften Ausgestaltung der Erfindung ist vorgesehen, dass der Grundkörper eine Länge von höchstens 5 mm, das heißt beispielsweise von 0,1 mm, 0,2 mm, 0,3 mm, 0,4 mm, 0,5 mm, 0,6 mm, 0,7 mm, 0,8 mm, 0,9 mm, 1,0 mm, 1,1 mm, 1,2 mm, 1,3 mm, 1,4 mm, 1,5 mm, 1,6 mm, 1,7 mm, 1,8 mm, 1,9 mm, 2,0 mm, 2,1 mm, 2,2 mm, 2,3 mm, 2,4 mm, 2,5 mm, 2,6 mm, 2,7 mm, 2,8 mm, 2,9 mm, 3,0 mm, 3,1 mm, 3,2 mm, 3,3 mm, 3,4 mm, 3,5 mm, 3,6 mm, 3,7 mm, 3,8 mm, 3,9 mm, 4,0 mm, 4,1 mm, 4,2 mm, 4,3 mm, 4,4 mm, 4,5 mm, 4,6 mm, 4,7 mm, 4,8 mm, 4,9 mm oder 5,0 mm, und/oder eine Dicke von höchstens 3 mm, das heißt beispielsweise von 0,1 mm, 0,2 mm, 0,3 mm, 0,4 mm, 0,5 mm, 0,6 mm, 0,7 mm, 0,8 mm, 0,9 mm, 1,0 mm, 1,1 mm, 1,2 mm, 1,3 mm, 1,4 mm, 1,5 mm, 1,6 mm, 1,7 mm, 1,8 mm, 1,9 mm, 2,0 mm, 2,1 mm, 2,2 mm, 2,3 mm, 2,4 mm, 2,5 mm, 2,6 mm, 2,7 mm, 2,8 mm, 2,9 mm oder 3,0 mm besitzt. Unter der Länge des Grundkörpers wird dabei seine Ausdehnung entlang seiner längsten Achse verstanden. Im Fall eines kreisrunden Grundkörpers entspricht seine Länge damit dem Durchmesser. Unter der Dicke des Grundkörpers wird dementsprechende seine maximale Ausdehnung senkrecht zur längsten Achse verstanden. Hierdurch kann der Grundkörper optimal an unterschiedliche Krankheitsbilder angepasst werden. Generell kann ein größerer bzw. voluminöserer Grundkörper mit entsprechend mehr Wirkstoff beladen werden als ein kleinerer bzw. weniger voluminöser. Umgekehrt kann der Grundkörper nicht beliebig groß gewählt werden, so dass die jeweils optimale Länge bzw. Dicke für jeden Patienten individuell von einem Arzt bestimmt werden sollte. Alternativ oder zusätzlich kann vorgesehen sein, dass der Grundkörper eine runde, ovale oder prismatische Form besitzt. Obwohl die Form des Grundkörpers generell nicht auf eine bestimmte Geometrie beschränkt ist, haben sich die genannten Formen für die meisten Anwendungsfälle und insbesondere für die Verwendung bei der Behandlung der feuchten Makuladegeneration als vorteilhaft gezeigt.

In einer weiteren vorteilhaften Ausgestaltung der Erfindung ist vorgesehen, dass der Grundkörper eine Makulaöffnung aufweist, welche vorzugsweise einen Durchmesser von mindestens 1 mm und/oder von höchstens 3 mm besitzt. Mit anderen Worten weist der Grundkörper eine Durchgangsöffnung oder Aussparung zur Anordnung über der Makula des betreffenden Patienten auf, wobei die Makulaöffnung einen Durchmesser bzw. eine Länge von 1,00 mm, 1,05 mm, 1,10 mm, 1,15 mm, 1,20 mm, 1,25 mm, 1,30 mm, 1,35 mm, 1,40 mm, 1,45 mm, 1,50 mm, 1,55 mm, 1,60 mm, 1,65 mm, 1,70 mm, 1,75 mm, 1,80 mm, 1,85 mm, 1,90 mm, 1,95 mm, 2,00 mm, 2,05 mm, 2,10 mm, 2,15 mm, 2,20 mm, 2,25 mm, 2,30 mm, 2,35 mm, 2,40 mm, 2,45 mm, 2,50 mm, 2,55 mm, 2,60 mm, 2,65 mm, 2,70 mm, 2,75 mm, 2,80 mm, 2,85 mm, 2,90 mm, 2,95 mm oder 3,00 mm aufweist. In Einzelfällen können grundsätzlich auch kleinere oder größere Durchmesser vorgesehen sein. Hierdurch kann eine Sehbehinderung des Patienten durch das auf die Retina aufgebrachte ophthalmologische Implantat vorteilhaft ausgeschlossen werden, ohne die antiangiogenetische Wirkung zu beeinträchtigen. Die Makulaöffnung ist vorzugsweise zentral im Grundkörper ausgebildet, kann in bestimmten Fällen aber auch exzentrisch ausgebildet sein.

In einer weiteren vorteilhaften Ausgestaltung der Erfindung ist das hydrophile Polymer ausgewählt aus einer Gruppe, die Alginsäure, Carboxymethylcellulose, Chitosan, Dextran, Dextransulfat, Pentosanpolysulfat, Carrageenan, Pektin, Pektinderivate, Cellulose, Cellulosederivate, Glucosaminoglykane, insbesondere Hyaluronsäure, Chondroitinsulfat, Dermatansulfat, Keratansulfat, Heparin, Heparansulfat, Hyaluronan, Agarose, Stärke, Methylcellulose, Polymannuronsäure, Polyguluronsäure, Polyglucuronsäure, Amylose, Amylopektin, Callose, Polygalactomannan, Xanthan, Poly(ethylenoxid), Poly(ethylenglykol), Kollagen, Gelatine, Fibrin, Fibrinogen, Fibronektin, Vitronectin, Poly(ethylenoxid), Poly(acrylsäure), Poly(methacrylsäure), Poly(acrylamid), Polyvinylpyrrolidon, Poly(aminosäuren); Poly(amine), Poly(imine), eine Mischung hieraus und/oder Copolymerisate hiervon und/oder pharmakologisch akzeptable Salze hiervon umfasst. Hierdurch können die Eigenschaften, beispielsweise die Viskoelastizität, optimal an den jeweiligen Einsatz- und Verwendungszweck des Implantats angepasst werden. Polysaccharide, die auch als Glykane bezeichnet werden, stellen generell eine Unterklasse der Kohlenhydrate dar und sind Vielfachzucker aus Monosaccharideinheiten (z. B. Glukose, Fruktose, Galactose usw.), welche eine Kette bilden. Jedes Monosaccharid, das auch als Einfachzucker bezeichnet wird, besteht aus einer Kette von Kohlenstoff-Atomen. Nach der Anzahl der Kohlenstoffatome spricht man von Triosen (3), Tetrosen (4), Pentosen (5), Hexosen (6), Heptosen (7) usw. Die Polysaccharide können nach Art ihrer Saccharideinheiten in Homoglykane mit nur einer Art Einfachzucker und Heteroglykane mit zwei oder mehr verschiedenen Arten von Einzelzuckern eingeteilt werden. Weiterhin können Polysaccharide unsubstituiert oder substituiert sein und eine oder mehrere Seitengruppen wie beispielsweise Hydroxyl-, Carboxy-, Amino- oder Sulfatgruppen tragen. Hyaluronsäure gehört zu der Gruppe der Glykosaminoglykane, bei denen es sich um Polysaccharide handelt, die aus repetitiven, linear aufgebauten und sauren Disaccharid-Einheiten bestehen. Die Disaccharid-Einheiten von Glykosaminoglykanen bestehen generell aus Estern einer Uronsäure. Meist handelt es sich um Glucuronsäure, seltener um Iduronsäure. Die Disaccharid-Einheiten sind 1-3-glykosidisch mit einem Aminozucker (z.B. N-Acetylglucosamin) verbunden. Die Kettenbildung der Disaccharid-Einheiten erfolgt durch 1-4-glykosidische Bindungen. Durch entsprechende Seitengruppen (Hydroxyl-, Carboxy- oder Sulfatgruppen) sind die Glykosaminoglykane negativ geladen. Glykosaminoglykane können in sulfatierte und nicht-sulfatierte Glykosaminoglykane eingeteilt werden. Hyaluronsäure (Hyaluronan) ist das einzige nicht-sulfatierte Glykosaminoglykan und weist freie Carboxy-Gruppen auf. In einer weiteren vorteilhaften Ausgestaltung der Erfindung ist vorgesehen, dass das hydrophile Polymer unvernetzt ist oder dass das das hydrophile Polymer selbstvernetzt und/oder mit wenigstens einem Quervernetzer quervernetzt ist. Hierdurch kann die biologische Stabilität und Abbaurate optimal an den jeweiligen Anwendungszweck angepasst werden. Generell wird die biologische Stabilität des wenigstens einen hydrophilen Polymers durch Quervernetzung erhöht. Unter einer Selbstvernetzung wird dabei eine gegebenenfalls aktivierte intra- und/oder intermolekulare Vernetzung von unterschiedlichen funktionellen Gruppen des Polymers unter Ausbildung eines dreidimensionalen Netzwerks verstanden. Alternativ oder zusätzlich kann wenigstens ein Quervernetzer (engl. cross-linker) mit mindestens zwei reaktiven Gruppen zur Quervernetzung des Polymers vorgesehen sein. Der Quervernetzer kann dabei zwei oder mehr gleiche reaktiven Gruppen (homofunktioneller Quervernetzer) oder zwei oder mehr unterschiedlichen Gruppen (heterofunktioneller Quervernetzer) aufweisen, die gegebenenfalls nach Aktivierung mit korrespondierenden funktionellen Gruppen des hydrophilen Polymers unter Ausbildung eines dreidimensionalen Netzwerks reagieren. Durch geeignete Wahl des Quervernetzers oder der Quervernetzer können verschiedene Eigenschaften des Implantats eingestellt werden, beispielsweise die biologische Abbaubarkeit bzw. Abbaugeschwindigkeit, die Formstabilität, die optischen Eigenschaften, die Beladbarkeit mit Wirkstoff usw.

Dabei hat es sich als vorteilhaft gezeigt, wenn der Quervernetzer ausgewählt ist aus einer Gruppe, die Glyoxal, 2,2'-Bioxiran, Polyethylenglykoldiamin, Divinylsulfon und 1,4-Butandioldiglycidylether umfasst. Beispielsweise kann Hyaluronsäure mit 2,2'-Bioxiran (1,2:3,4-Diepoxybutan) vernetzt werden, wobei zusätzlich Natriumborhydrid als Aktivator und Unterstützer der Quervernetzungsreaktion verwendet werden kann, der zusätzlich überschüssiges 2,2'-Bioxiran inaktiviert, so dass keine unerwünschten Ausgangsprodukte zurückbleiben. Alternativ oder zusätzlich ist eine Umsetzung von Hyaluronsäure und anderen Polymeren mit Hydroxygruppen mit Divinylsulfon möglich, wodurch über eine Michael-Addition die Ausbildung von Etherbrücken realisierbar ist, was zu besonders langzeitstabilen Polymeren führt. Unter ähnlichen Bedingungen wie bei der Selbstvernetzung können Hyaluronsäure und andere Polymeren mit Carbonsäuregruppen auch mit Polyethylenglykoldiamin (PEG-Diamin) vernetzt werden.

In einer weiteren vorteilhaften Ausgestaltung der Erfindung ist vorgesehen, dass das hydrophile Polymer Alginsäure und/oder ein Alginat umfasst und durch Metallionen, insbesondere durch Calcium-Ionen komplexiert ist. Alginsäure ist ein Polysaccharid, das von Braunalgen und von einigen Bakterien gebildet wird und aus den beiden Uronsäuren α-L-Guluronsäure (GulUA) und β-D-Mannuronsäure (ManUA) besteht, welche 1,4-glycosidisch in wechselndem Verhältnis zu linearen Ketten verbunden sind. Die Salze der Alginsäure werden als Alginate bezeichnet. Alginate bilden homopolymere Bereiche, in denen Mannuronsäure oder Guluronsäure als Blöcke vorliegen. Diese Blöcke werden als GG- oder MM-Blöcke bezeichnet und liegen in einer Art Faltstruktur vor. Insbesondere die GG-Blöcke bilden eine regelmäßige Zickzack-Struktur. Durch Einlagerung von Metallionen in die Zickzackstruktur der GG-Blöcke kommt es zu einer Gelierung, da sich Zickzackstrukturen anderer Alginate an diese anlagern, wodurch dreidimensionale Strukturen in der Art einer Vernetzung gebildet werden. Da Metallionen wie beispielsweise Calcium in diesen Strukturen wie Eier in der Schachtel liegen, wird dieses Modell auch als "Eierschachtel-Modell" (englisch "Eggbox-model") bezeichnet. Weil die Reaktion insbesondere mit Calcium sehr schnell erfolgt, können verschiedene Strategien vorgesehen sein, um diese "Vernetzungsreaktion" bzw. Gelierung langsamer und kontrollierter ablaufen zu lassen. Hierzu können insbesondere schwerlösliche Metallsalze verwendet werden, die mittels Säuerung die komplexierenden Metallionen freisetzen. Über den zeitlichen Verlauf und den pH-Endwert der Säuerung kann die Geschwindigkeit der Gelierung gesteuert werden. Neben Calcium (Ca²⁺) können auch andere divalente Kationen, beispielsweise Mg²⁺, Ba²⁺, Cu²⁺, Fe²⁺ oder Zn²⁺, einzeln und in beliebiger Kombination, zur Gelierung verwendet werden.

In einer weiteren vorteilhaften Ausgestaltung der Erfindung ist vorgesehen, dass der wenigstens eine Angiogenesehemmer am Grundkörper immobilisiert ist. Hierdurch kann die Verweilzeit des Angiogenesehemmers an einer Stelle, an der eine Hemmung der Neovaskularisierung im Verhältnis zur Verweilzeit des Bindungsteils ohne das hydrophile Polymer erwünscht ist, signifikant gesteigert werden.

Weitere Vorteile ergeben sich dadurch, dass der wenigstens eine Angiogenesehemmer kovalent an das wenigstens eine hydrophile Polymer gebunden ist und/oder dass das wenigstens eine hydrophile Polymer und der wenigstens eine Angiogenesehemmer als interpenetrierendes Netzwerk vorliegen. Durch eine kovalente Anbindung kann die Wirkung des Angiogenesehemmers besonders langanhaltend sichergestellt werden. Wenn das hydrophile Polymer biologisch abbaubar ist, kann der kovalent angebundene Angiogenesehemmer über die Abbaurate des Polymers kontrolliert abgegeben werden. Ähnliches kann mit Hilfe eines interpenetrierenden Netzwerks bewerkstelligt werden. Interpenetrierende Netzwerke (IPN) bestehen aus zwei oder mehr Netzwerken, die sich gegenseitig durchdringen. Daher werden sie auch Durchdringungsnetzwerke genannt. Die wenigstens zwei Netzwerkkomponenten können grundsätzlich chemisch identisch oder verschieden sein. Eine der wenigstens zwei Netzwerkkomponenten ist das hydrophile Polymer, während eine andere der wenigstens zwei Netzwerkkomponenten den Angiogenesehemmer umfasst, wobei der Angiogenesehemmer in diesem Fall beispielsweise kovalent an ein gleiches hydrophiles oder an ein unterschiedliches hydrophiles oder hydrophobes Polymer gebunden sein kann, oder aus dem Angiogenesehemmer besteht. Der Angiogenesehemmer kann je nach Ausgestaltung des interpenetrierenden Netzwerks durch biologischen Abbau der ersten Netzwerkkomponente (d.h. des hydrophilen Polymers) und/oder als mobile Komponente durch Diffusion aus der ersten Netzwerkkomponente kontrolliert freigesetzt werden. Gleiches gilt natürlich für gegebenenfalls vorhandene weitere Wirkstoffe, mit denen der Grundkörper beladen sein kann.

Dabei hat es sich weiterhin als vorteilhafte gezeigt, wenn die wenigstens eine Trägerfolie zumindest überwiegend aus einem Polyamid und/oder aus einem Polyimid und/oder aus Polylactid-co-Glycolid (PLGA) besteht. Hierdurch kann die Trägerfolie einerseits biokompatibel und andererseits biologisch abbaubar ausgestaltet werden.

Ein zweiter Aspekt der Erfindung betrifft ein Implantationswerkzeug, welches eine Ladekammer umfasst, in welcher wenigstens ein ophthalmologisches Implantat gemäß dem ersten Erfindungsaspekt zur Implantation auf eine Retina eines Auges eines menschlichen oder tierischen Patienten angeordnet ist. Hierdurch kann das Implantat zur Behandlung von Augenkrankheiten, welche Angiogenese umfassen, und insbesondere zur Behandlung der feuchten Makuladegeneration beispielsweise über den Bereich der Pars plana in ein Patientenauge eingeführt und im Bereich der Makula auf die Retina aufgesetzt werden, wodurch der Angiogenesehemmer nicht im gesamten Glaskörper verteilt wird, sondern gezielt an der Stelle lokalisiert werden kann, wo unerwünschtes Gefäßwachstum stattfindet. In manchen Ausgestaltungen enthält die Ladekammer zusätzlich ein geeignetes und biokompatibles Gleitmittel, beispielsweise Hyaluronsäure. Weitere Merkmale und deren Vorteile sind den Beschreibungen des ersten Erfindungsaspekts zu entnehmen, wobei vorteilhafte Ausgestaltungen des ersten Erfindungsaspekts als vorteilhafte Ausgestaltungen des zweiten Erfindungsaspekts und umgekehrt anzusehen sind.

In einer vorteilhaften Ausgestaltung der Erfindung besitzt das Implantationswerkzeug eine Injektorspitze mit einer Länge von mindestens 18 mm und/oder von höchstens 30 mm. Beispielsweise kann die Injektorspitze eine Länge von 18,0 mm, 18,5 mm, 19,0 mm, 19,5 mm, 20,0 mm, 20,5 mm, 21,0 mm, 21,5 mm, 22,0 mm, 22,5 mm, 23,0 mm, 23,5 mm, 24,0 mm, 24,5 mm, 25,0 mm, 25,5 mm, 26,0 mm, 26,5 mm, 27,0 mm, 27,5 mm, 28,0 mm, 28,5 mm, 29,0 mm, 29,5 mm oder 30,0 mm aufweisen, wobei im Einzelfall auch kürzere oder längere Dimensionen vorgesehen sein können. Hierdurch kann das Implantat besonders gut insbesondere in menschliche Augen eingesetzt werden, die üblicherweise einen Durchmesser zwischen etwa 20 mm und etwa 25 mm besitzen. Alternativ oder zusätzlich kann die Injektorspitze einen Innendurchmesser von mindestens 0,8 mm und/oder von höchstens 2,5 mm, also beispielsweise von 0,8 mm, 0,9 mm, 1,0 mm, 1,1 mm, 1,2 mm, 1,3 mm, 1,4 mm, 1,5 mm, 1,6 mm, 1,7 mm, 1,8 mm, 1,9 mm, 2,0 mm, 2,1 mm, 2,2 mm, 2,3 mm, 2,4 mm oder 2,5 mm besitzen. Hierdurch können erfindungsgemäße Implantate mit einer möglichst geringen Beeinträchtigung des Patientenauges durch Inzisionen von weniger als 4 mm Länge, beispielsweise mit Inzisionen zwischen etwa 2 mm und etwa 3 mm eingesetzt werden, wobei die genannten Innendurchmesser dem Umstand Rechnung tragen, dass das erfindungsgemäße Implantat einerseits nicht beliebig klein gefaltet oder gerollt werden kann und andererseits eine gewisse Mindeststeifigkeit besitzen sollte, um bei der Implantation nicht beschädigt zu werden.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: eine schematische Darstellung eines erfindungsgemäßen ophthalmologischen Implantats;
- Fig. 2: eine schematische und teilgeschnittene Perspektivansicht eines menschlichen Auges, in welches das erfindungsgemäße Implantat im Bereich der Retina eingesetzt wird;
- Fig. 3: eine schematische und teilgeschnittene Perspektivansicht des Auges mit eingesetztem Implantat;
- Fig. 4: eine schematische anatomische Perspektivansicht der Retina mit dem aufgesetzten erfindungsgemäßen Implantat;
- Fig. 5: eine schematische anatomische Perspektivansicht der Retina und des erfindungsgemäßen Implantats.

### Bevorzugte Ausführung der Erfindung

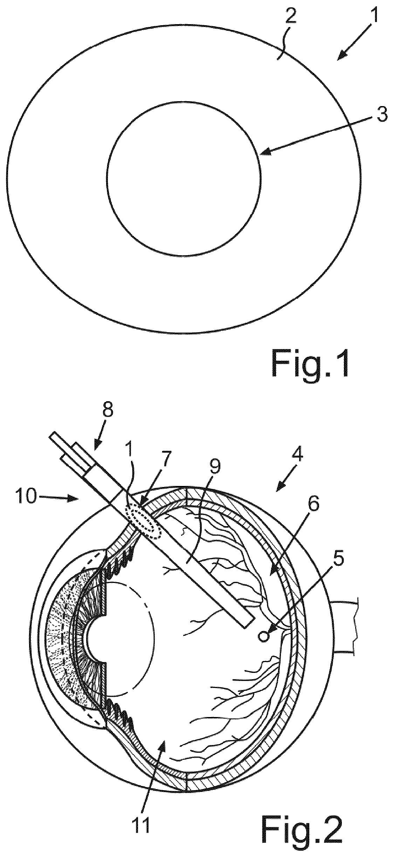

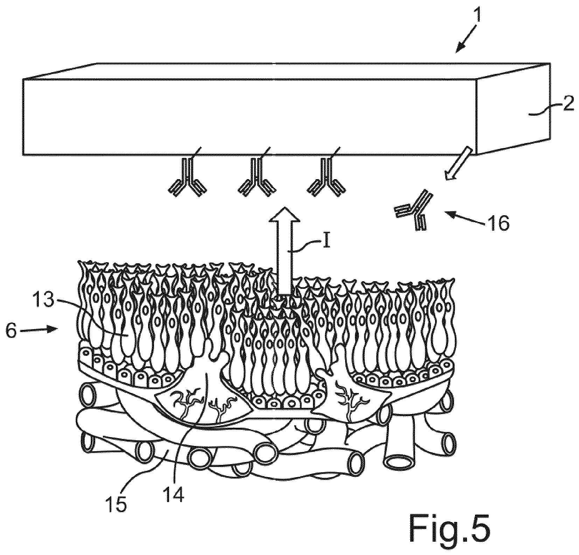

Fig. 1 zeigt eine schematische Darstellung eines erfindungsgemäßen ophthalmologischen Implantats 1, welches zur Behandlung der feuchten Makuladegeneration verwendet werden kann. Das ophthalmologische Implantat 1 umfasst im gezeigten Ausführungsbeispiel einen kreisringförmigen Grundkörper 2, der aus einem hydrophilen Polymer besteht, an welches ein Angiogenesehemmer 16 (Fig. 5) kovalent angebunden ist. Der Grundkörper 2 besitzt einen Durchmesser von etwa 2,5 mm und eine zentral angeordnete, ebenfalls runde Makulaöffnung 3, die einen Durchmesser von etwa 1,5 mm besitzt. Die Dimensionen sowie die Geometrien des Grundkörpers 2 und der Makulaöffnung 3 können in Abhängigkeit der individuellen Gegebenheiten des Auges 4 (Fig. 2), in welches das Implantat 1 eingesetzt werden soll, variiert werden. Beispielsweise kann der Grundkörper 2 auch oval oder prismatisch ausgebildet sein. Die Größe und Form der Makulaöffnung 3 sollte generell auf die Größe und Form der Makula 5 des Auges 4 abgestimmt sein, um Sehbeeinträchtigungen des Patienten zu vermeiden.

Das hydrophile Polymer erhöht die Verweilzeit des Angiogenesehemmers 16, bei welchem es sich beispielsweise um einen neovaskularisationshemmenden Antikörper handeln kann, an einer Stelle, an der eine Hemmung der Neovaskularisierung im Verhältnis zur Verweilzeit des Angiogenesehemmers 16 ohne das hydrophile Polymer erwünscht und notwendig ist. Bei dem hydrophilen Polymer kann es sich beispielsweise um Hyaluronsäure (HA) mit der allgemeinen Formel 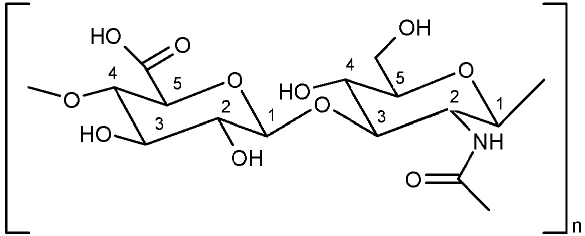 handeln. Saure Polysaccharide und ihre entsprechenden Salze (z. B. Hyaluronat oder Alginat) können leicht mit Aminogruppen von Aminosäureseitenketten eines Proteins/Antikörpers konjugiert werden. Hierzu werden die Carbonsäuregruppen durch Aktivierung mit N-Hydroxysulfosuccinimid (Sulfo-NHS) mit der Formel 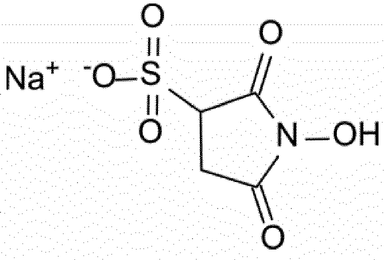 über eine durch 1-Ethyl-3-(3-dimethylaminopropyl)carbodiimid (EDC) mit der Formel 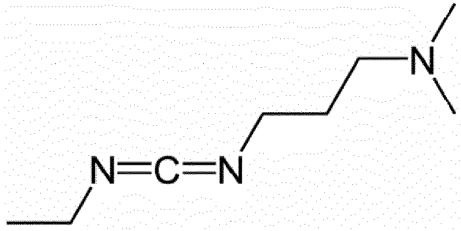 und 4-Dimethylaminophenol (4-DMPA) mit der Formel 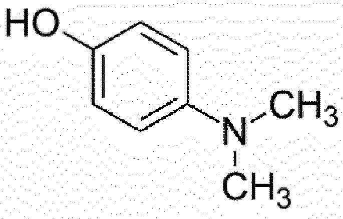 vermittelte Reaktion mit einem antiangiogenetisch wirksamen Protein/Antikörper gekoppelt:

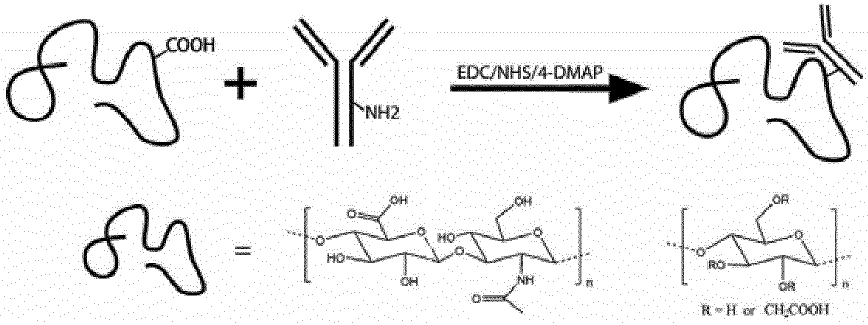

Anstelle von Hyaluronsäure kann beispielsweise auch Carboxymethylcellulose (CMC) oder ein anderes hydrophiles Polymer mit Caroxylgruppen verwendet werden. Die allgemeine Reaktionsführung ist in Sun et al. (Cytokine Binding by Polysaccharide-Antibody Conjugates, Mol Pharm. 2010 October 4; 7(5): 1769-1777) beschrieben. Generell können aber auch andere hydrophile Polymere, alternative Angiogenesehemmer 16 und abweichende Kopplungsreaktionen vorgesehen sein.

Das hydrophile Polymer kann in Abhängigkeit der vorhandenen funktionellen Gruppen z. B. durch 2,2'-Bioxiran (1,2:3,4-Diepoxybutan) oder 1,4-Butandioldiglycidylether oder durch einen anderen geeigneten Quervernetzer vernetzt werden. Über die Vernetzungsrate können insbesondere die mechanischen Eigenschaften, die biologische Abbaubarkeit und die Haftung des Implantats 1 an der Retina 6 gesteuert werden. Neben Polysacchariden wie Hyaluronsäure können auch Chitosan oder Carboxymethylcellulose mit 2,2'-Bioxiran oder 1,4-Butandiol-diglycidylether quervernetzt werden. Wenn Alginsäure bzw. ein Alginat als hydrophiles Polymer verwendet wird, kann die Steifigkeit des Implantats 1 auch durch Gelierung z. B. mit Calciumionen kontrolliert werden.

Zur Vernetzung von Hyaluronsäure mit 2,2'-Bioxiran (1,2:3,4-Diepoxybutan) werden beispielsweise 100 mg Natriumhyaluronat (team-pharma Volkmar Lippold GmbH) in 2 ml 0,2 M NaOH gelöst, und anschließend zunächst Natriumborhydrid und dann das 2,2'-Bioxiran in unterschiedlichen Mengen, bezogen auf die Natronlauge, unter starkem Rühren zugesetzt. Der Anteil dieses Quervernetzers lag zwischen 40 Vol.-% und 90 Vol.-%, beispielsweise bei 80 Vol.-%, wodurch der Quervernetzungsgrad und damit die Materialeigenschaften des resultierenden quervernetzten Polymers variiert werden können. Diese Mischung wird für 2 h bei 50 °C gerührt, wobei nach ca. 75 min eine GelBildung durch Klümpchen angezeigt wird. Zum Ende der Reaktion wird der gebildete Gel-Ball mit einem Spatel zerkleinert, die Stückchen mit etwas Wasser weiter gequollen und zwischen zwei silanisierten Glasplatten mit einem Spacer von 1 mm fein verteilt und bis zu 48 h lang gepresst, wodurch ein homogenes Gel entsteht. Anschließend wird die eine Glasplatte entfernt und das Gel durch wiederholtes Benetzen mit Wasser bzw. BSS (PURI CLEAR) und Entfernen des Überstands bis zur Geruchsneutralität gewaschen.

Danach wird eine 0,05 mm dicke Polyimidfolie aufgebracht, die Glasplatte wieder aufgelegt und das Gel von der anderen Seite auf gleiche Art gewaschen.

Die Sterilisation der Gele erfolgt durch Dampfsterilisation für 20 min bei 121°C in einem Sterisafe A4-Sterilgut-Container.

Der wenigstens eine antiangiogenetisch wirksame Antikörper wird vorzugsweise kovalent an das hydrophile Polymer gebunden, um die Diffusion des Antikörpers von der Stelle, wo er benötigt wird, zu verhindern. So verteilt beispielsweise eine herkömmliche Anti-VEGF-Injektion (Ranibizumab/Lucentis^{®}) in den Glaskörper die Antikörpermoleküle im gesamten Glaskörperraum, obwohl diese nur im Bereich der Makula 5 benötigt werden, wo unerwünschtes Gefäßwachstum stattfindet. Das erfindungsgemäße Implantat 1 kann demgegenüber den oder die Angiogenesehemmer 16 unmittelbar in der Nähe der Makula 5 lokalisieren bzw. immobilisieren. Dadurch kann neben einer verbesserten und länger anhaltenden Wirkung auch die Menge kostenintensiver Antikörper auf ein therapeutisch sinnvolles Minimum reduziert werden. Alternativ oder zusätzlich zu einer kovalenten Kopplung kann auch ein interpenetrierendes Netzwerk aus hydrophilem Polymer als erster Netzwerkkomponente und Angiogenesehemmer 16 als zweiter Netzwerkkomponente vorgesehen sein, wobei der Angiogenesehemmer 16 gegebenenfalls kovalent an den gleichen hydrophilen Polymertyp oder an ein abweichendes hydrophiles oder hydrophobes Polymer gekoppelt sein kann. Weiterhin kann generell vorgesehen sein, dass der Grundkörper 2 mit einem oder mehreren weiteren Wirkstoffen beladen ist, wobei der wenigstens eine weitere Wirkstoff in das hydrophile Polymer eingelagert und/oder ebenfalls kovalent angebunden sein kann. Hierdurch kann das Implantat 1 als Verabreichungssystem für weitere Medikamente fungieren und zur gleichzeitigen Behandlung unterschiedlicher Krankheiten oder Symptome verwendet werden.

Fig. 2 zeigt eine schematische und teilgeschnittene Perspektivansicht eines menschlichen Auges 4, in welches das erfindungsgemäße Implantat 1 im Bereich der Retina 6 eingesetzt wird. Hierzu ist das Implantat 1 aufgerollt in einer Ladekammer 7 eines Implantationswerkzeug 8 angeordnet. Eine an die Ladekammer 7 anschließende Injektorspitze 9 des Implantationswerkzeugs 8 wird dann durch die Wand des Auges 4 auf Höhe der Region der "Pars plana" 10 in das Auge 4 eingeführt, um das Implantat 1 auf die Retina 6 aufzubringen. Die Injektorspitze 9 besitzt hierzu eine Länge von etwa 28 mm sowie einen Durchmesser von etwa 1,2 mm, wobei auch andere Dimensionen vorgesehen sein können. Vorzugsweise enthält das Implantationswerkzeug 8 zusätzlich ein verträgliches Gleitmittel wie beispielsweise Hyaluronsäure. Für chirurgisches Werkzeug (Vitrektom, Scheren, Greifer, Häkchen etc.) kann bedarfsweise ein weiterer Zugang angelegt werden. Der Glaskörper 11 kann durch die Vitrektomie entfernt werden. Durch eine Infusion kann der Druck im Auge aufrechterhalten werden. Um die Haftung des Implantats 1 an der Retina 6 zu verbessern, kann der Glaskörperraum 11 beispielsweise mit einem Gas (z.B. SF₆), mit Silikonöl oder mit (Per) Fluorkohlenwasserstoff(en) gefüllt werden.

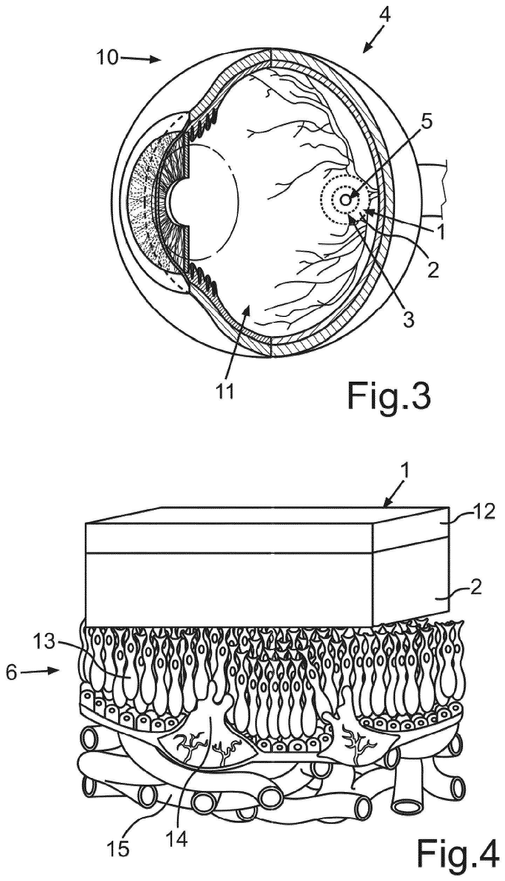

Fig. 3 zeigt eine schematische und teilgeschnittene Perspektivansicht des Auges 4 mit eingesetztem Implantat 1. Man erkennt, dass die Makulaöffnung 3 des Implantats 1 die Makula 5 ausspart, während der Grundkörper 2 des Implantats 1 als eine Art Pflaster oder Patch nicht nur etwaige Netzhautrisse versiegelt, sondern auch den Angiogenesehemmer 16 zur Hemmung der Neovaskularisierung im relevanten Bereich des Auges 4 lokalisiert und gegebenenfalls immobilisiert.

Fig. 4 zeigt eine schematische anatomische Perspektivansicht der Retina 6 mit dem aufgesetzten erfindungsgemäßen Implantat 1. Man erkennt, dass das Implantat 1, das aufgrund des hydrophilen Polymers auf der Netzhautoberfläche 13 haftet, erfindungsgemäß eine vorzugsweise transparente und/oder biologisch abbaubare Trägerfolie 12 aufweist, die auf einer von der Retina 6 abgewandten Seite des Grundkörpers 2 aufgebracht ist, um den Grundkörper 2 zu stabilisieren. Die Trägerfolie 12 kann beispielsweise aus biologisch abbaubarem Polylactid-co-Glycolid oder aus einem Polyimid bestehen und besitzt im gezeigten Beispiel eine Dicke von etwa 0,05 mm.

Bei der feuchten Makuladegeneration tritt eine Ödembildung 14 unter der Retina 6 auf. Dabei wachsen Blutgefäße 15 in die Retina 6 ein. Diese Blutgefäße 15 bezeichnet man als choroidale Neovaskularisationen, kurz CNV, die zu einem starken Sehschärfeverlust führen können.

Fig. 5 zeigt eine schematische anatomische Perspektivansicht der Retina 6 und des erfindungsgemäßen Implantats 1. Das Implantat 1 ist dabei lediglich zur Verdeutlichung des Wirkprinzips von der Retina 6 abgehoben dargestellt. Man erkennt die an der Oberfläche des Grundkörpers 2 kovalent angebundenen Angiogenesehemmer 16, bei denen es sich um Antikörper handelt, die den gemäß Pfeil I abgegebenen Wachstumsfaktor VEGF binden und dadurch die Angiogenese hemmen.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, Einwaagefehlern und dergleichen - als vom Rahmen der Erfindung mitumfasst anzusehen.

### Bezugszeichenliste

- 1: Implantat
- 2: Grundkörper
- 3: Makulaöffnung
- 4: Auge
- 5: Makula
- 6: Retina
- 7: Ladekammer
- 8: Implantationswerkzeug
- 9: Injektorspitze
- 10: Pars plana
- 11: Glaskörper
- 12: Trägerfolie
- 13: Netzhautoberfläche
- 14: Ödembildung
- 15: Blutgefäße
- 16: Angiogenesehemmer

## Patentansprüche

1. Ophthalmologisches Implantat (1) zur Verwendung bei der Behandlung einer Augenkrankheit, welche Angiogenese umfasst, insbesondere zur Behandlung der feuchten Makuladegeneration, wobei das ophthalmologische Implantat (1) einen im Bereich einer Retina (6) eines menschlichen oder tierischen Patienten anzuordnenden Grundkörper (2), der zumindest teilweise aus wenigstens einem hydrophilen Polymer besteht, und einen am Grundkörper (2) angeordneten Angiogenesehemmer (16) umfasst, **dadurch gekennzeichnet, dass** wenigstens eine Trägerfolie (12) auf zumindest einem Teil einer Oberfläche des Grundkörpers (2) aufpolymerisiert ist, um den Grundkörper (2) zu stabilisieren, wobei die wenigstens eine Trägerfolie (12) auf einer von der Retina (6) abzuwendenden Seite des Grundkörpers (2) auf diesen aufgebracht ist, und wobei der wenigstens eine Angiogenesehemmer (16) ausgewählt ist aus einer Gruppe, die Bevacizumab, Brolucizumab, Ranibizumab, Ramucirumab, Aflibercept, Pegaptanib, Thalidomid, Axitinib, Lenvatinib, Lucitanib, Motesanib, Pazopanib, Regorafenib, Sorafenib, Sunitinib, Tivozanib, Vatalanib und Biosimilars davon umfasst.

2. Ophthalmologisches Implantat (1) nach Anspruch 1,
**dadurch gekennzeichnet, dass**
der Grundkörper (2) eine Länge von höchstens 5 mm und/oder eine Dicke von höchstens 3 mm besitzt und/oder dass der Grundkörper (2) eine runde, ovale oder prismatische Form besitzt.

3. Ophthalmologisches Implantat (1) nach Anspruch 1 oder 2,
**dadurch gekennzeichnet, dass**
der Grundkörper (2) eine Makulaöffnung (3) aufweist, welche vorzugsweise einen Durchmesser von mindestens 1 mm und/oder von höchstens 3 mm besitzt.

4. Ophthalmologisches Implantat (1) nach einem der Ansprüche 1 bis 3,
**dadurch gekennzeichnet, dass**
das hydrophile Polymer ausgewählt ist aus einer Gruppe, die Alginsäure, Carboxymethylcellulose, Chitosan, Dextran, Dextransulfat, Pentosanpolysulfat, Carrageenan, Pektin, Pektinderivate, Cellulose, Cellulosederivate, Glucosaminoglykane, insbesondere Hyaluronsäure, Chondroitinsulfat, Dermatansulfat, Keratansulfat, Heparin, Heparansulfat, Hyaluronan, Agarose, Stärke, Methylcellulose, Polymannuronsäure, Polyguluronsäure, Polyglucuronsäure, Amylose, Amylopektin, Callose, Polygalactomannan, Xanthan, Poly(ethylenoxid), Poly(ethylenglykol), Kollagen, Gelatine, Fibrin, Fibrinogen, Fibronektin, Vitronectin, Poly(ethylenoxid), Poly(acrylsäure), Poly(methacrylsäure), Poly(acrylamid), Polyvinylpyrrolidon, Poly(aminosäuren); Poly(amine), Poly(imine), eine Mischung hieraus und/oder Copolymerisate hiervon und/oder pharmakologisch akzeptable Salze hiervon umfasst.

5. Ophthalmologisches Implantat (1) nach einem der Ansprüche 1 bis 4,
**dadurch gekennzeichnet, dass**
das hydrophile Polymer unvernetzt ist oder dass das das hydrophile Polymer selbstvernetzt und/oder mit wenigstens einem Quervernetzer quervernetzt ist.

6. Ophthalmologisches Implantat (1) nach Anspruch 5,
**dadurch gekennzeichnet, dass**
der Quervernetzer ausgewählt ist aus einer Gruppe, die Glyoxal, 2,2'-Bioxiran, Polyethylenglykoldiamin, Divinylsulfon und 1,4-Butandioldiglycidylether umfasst.

7. Ophthalmologisches Implantat (1) nach einem der Ansprüche 1 bis 6,
**dadurch gekennzeichnet, dass**
das hydrophile Polymer Alginsäure und/oder ein Alginat umfasst und durch Metallionen, insbesondere durch Calcium-Ionen komplexiert ist.

8. Ophthalmologisches Implantat (1) nach einem der Ansprüche 1 bis 7,
**dadurch gekennzeichnet, dass**
der wenigstens eine Angiogenesehemmer (16) am Grundkörper (2) immobilisiert ist.

9. Ophthalmologisches Implantat (1) nach Anspruch 8,
**dadurch gekennzeichnet, dass**
der wenigstens eine Angiogenesehemmer (16) kovalent an das wenigstens eine hydrophile Polymer gebunden ist und/oder dass das wenigstens eine hydrophile Polymer und der wenigstens eine Angiogenesehemmer (16) als interpenetrierendes Netzwerk vorliegen.

10. Ophthalmologisches Implantat (1) nach einem der Ansprüche 1 bis 9,
**dadurch gekennzeichnet, dass**
die wenigstens eine Trägerfolie (12) zumindest überwiegend aus einem Polyamid und/oder aus einem Polyimid und/oder aus Polylactid-co-Glycolid besteht.

11. Implantationswerkzeug (8), welches eine Ladekammer (7) umfasst, in welcher wenigstens ein ophthalmologisches Implantat (1) nach einem der Ansprüche 1 bis 10 zur Implantation auf eine Retina (6) eines Auges (4) eines menschlichen oder tierischen Patienten angeordnet ist.

12. Implantationswerkzeug (8) nach Anspruch 11,
**dadurch gekennzeichnet, dass**
dieses eine Injektorspitze (9) mit einer Länge von mindestens 18 mm und/oder von höchstens 30 mm und/oder eine Injektorspitze (9) mit einem Innendurchmesser von mindestens 0,8 mm und/oder von höchstens 2,5 mm besitzt.

## Claims

1. Ophthalmological implant (1) for use in the treatment of an eye disease comprising angiogenesis, in particular for the treatment of wet macular degeneration, the ophthalmological implant (1) comprising a main body (2) which is to be arranged in the region of a retina (6) of a human or animal patient and at least partly consists of at least one hydrophilic polymer and comprising an angiogenesis inhibitor (16) arranged on the main body (2), **characterized in that** at least one support film (12) is polymerized on at least a portion of a surface of the main body (2) in order to stabilize the main body (2), the at least one support film (12) being applied to the main body (2) on a side thereof facing away from the retina (6), and the at least one angiogenesis inhibitor (16) being selected from a group comprising bevacizumab, brolucizumab, ranibizumab, ramucirumab, aflibercept, pegaptanib, thalidomide, axitinib, lenvatinib, lucitanib, motesanib, pazopanib, regorafenib, sorafenib, sunitinib, tivozanib, vatalanib and biosimilars thereof.

2. Ophthalmological implant (1) according to Claim 1, **characterized in that**
the main body (2) has a length of at most 5 mm and/or a thickness of at most 3 mm and/or **in that** the main body (2) has a round, oval or prismatic shape.

3. Ophthalmological implant (1) according to Claim 1 or 2,
**characterized in that**
the main body (2) has a macular opening (3) preferably having a diameter of at least 1 mm and/or of at most 3 mm.

4. Ophthalmological implant (1) according to any of Claims 1 to 3,
**characterized in that**
the hydrophilic polymer is selected from a group comprising alginic acid, carboxymethylcellulose, chitosan, dextran, dextran sulfate, pentosan polysulfate, carrageenan, pectin, pectin derivatives, cellulose, cellulose derivatives, glucosaminoglycans, especially hyaluronic acid, chondroitin sulfate, dermatan sulfate, keratan sulfate, heparin, heparan sulfate, hyaluronan, agarose, starch, methylcellulose, polymannuronic acid, polyguluronic acid, polyglucuronic acid, amylose, amylopectin, callose, polygalactomannan, xanthan, poly(ethylene oxide), poly(ethylene glycol), collagen, gelatin, fibrin, fibrinogen, fibronectin, vitronectin, poly(ethylene oxide), poly(acrylic acid), poly(methacrylic acid), poly(acrylamide), polyvinylpyrrolidone, poly(amino acids); poly(amines), poly(imines), a mixture thereof and/or copolymers thereof and/or pharmacologically acceptable salts thereof.

5. Ophthalmological implant (1) according to any of Claims 1 to 4,
**characterized in that**
the hydrophilic polymer is not crosslinked or **in that** the hydrophilic polymer is self-crosslinked and/or is crosslinked with at least one crosslinker.

6. Ophthalmological implant (1) according to Claim 5, **characterized in that**
the crosslinker is selected from a group comprising glyoxal, 2,2'-bioxirane, polyethylene glycol diamine, divinyl sulfone and 1,4-butanediol diglycidyl ether.

7. Ophthalmological implant (1) according to any of Claims 1 to 6,
**characterized in that**
the hydrophilic polymer comprises alginic acid and/or an alginate and is complexed by metal ions, especially by calcium ions.

8. Ophthalmological implant (1) according to any of Claims 1 to 7,
**characterized in that**
the at least one angiogenesis inhibitor (16) is immobilized on the main body (2).

9. Ophthalmological implant (1) according to Claim 8, **characterized in that**
the at least one angiogenesis inhibitor (16) is covalently bonded to the at least one hydrophilic polymer and/or **in that** the at least one hydrophilic polymer and the at least one angiogenesis inhibitor (16) are in the form of an interpenetrating network.

10. Ophthalmological implant (1) according to any of Claims 1 to 9,
**characterized in that**
the at least one support film (12) at least predominantly consists of a polyamide and/or of a polyimide and/or of polylactide-co-glycolide.

11. Implantation tool (8) comprising a loading chamber (7) in which at least one ophthalmological implant (1) according to any of Claims 1 to 10 is arranged for implantation onto a retina (6) of an eye (4) of a human or animal patient.

12. Implantation tool (8) according to Claim 11, **characterized in that**
it has an injector tip (9) having a length of at least 18 mm and/or of at most 30 mm and/or an injector tip (9) having an internal diameter of at least 0.8 mm and/or of at most 2.5 mm.

## Revendications

1. Implant ophtalmologique (1) destiné à être utilisé dans le traitement d'une maladie oculaire impliquant une angiogenèse, notamment dans le traitement de la dégénérescence maculaire humide, l'implant ophtalmologique (1) comprenant un corps de base (2) destiné à être agencé dans la région d'une rétine (6) d'un patient humain ou animal, qui est constitué au moins en partie d'au moins un polymère hydrophile, et un inhibiteur d'angiogenèse (16) agencé sur le corps de base (2), **caractérisé en ce qu'**au moins une feuille de support (12) est polymérisée sur au moins une partie d'une surface du corps de base (2) afin de stabiliser le corps de base (2), l'au moins une feuille de support (12) étant appliquée sur le corps de base (2) sur un côté de celui-ci détourné de la rétine (6), et l'au moins un inhibiteur d'angiogenèse (16) étant choisi parmi un groupe comprenant le bevacizumab, le brolucizumab, le ranibizumab, le ramucirumab, l'aflibercept, le pegaptanib, le thalidomide, l'axitinib, le lenvatinib, le lucitanib, le motesanib, le pazopanib, le regorafenib, le sorafenib, le sunitinib, le tivozanib, le vatalanib et leurs biosimilaires.

2. Implant ophtalmologique (1) selon la revendication 1,
**caractérisé en ce que**
le corps de base (2) a une longueur d'au plus 5 mm et/ou une épaisseur d'au plus 3 mm et/ou le corps de base (2) a une forme ronde, ovale ou prismatique.

3. Implant ophtalmologique (1) selon la revendication 1 ou 2,
**caractérisé en ce que**
le corps de base (2) présente une ouverture maculaire (3) qui a de préférence un diamètre d'au moins 1 mm et/ou d'au plus 3 mm.

4. Implant ophtalmologique (1) selon l'une quelconque des revendications 1 à 3,
**caractérisé en ce que**
le polymère hydrophile est choisi parmi un groupe comprenant l'acide alginique, la carboxyméthylcellulose, le chitosane, le dextrane, le sulfate de dextrane, le pentosane polysulfate, le carraghénane, la pectine, les dérivés de pectine, la cellulose, les dérivés de cellulose, les glucosaminoglycanes, notamment l'acide hyaluronique, le sulfate de chondroïtine, le sulfate de dermatane, le sulfate de kératane, l'héparine, le sulfate d'héparane, l'hyaluronane, l'agarose, l'amidon, la méthylcellulose, l'acide polymannuronique, l'acide polyguluronique, l'acide polyglucuronique, l'amylose, l'amylopectine, la callose, le polygalactomannane, le xanthane, le poly(oxyde d'éthylène), le poly(éthylène glycol), le collagène, la gélatine, la fibrine, le fibrinogène, la fibronectine, la vitronectine, le poly(oxyde d'éthylène), le poly(acide acrylique), le poly(acide méthacrylique), le poly(acrylamide), la polyvinylpyrrolidone, les poly(aminoacides) ; les poly(amines), les poly(imines), un mélange de ceux-ci et/ou des copolymères de ceux-ci et/ou des sels pharmacologiquement acceptables de ceux-ci.

5. Implant ophtalmologique (1) selon l'une quelconque des revendications 1 à 4,
**caractérisé en ce que**
le polymère hydrophile est non réticulé ou **en ce que** le polymère hydrophile est autoréticulé et/ou réticulé avec au moins un agent de réticulation.

6. Implant ophtalmologique (1) selon la revendication 5,
**caractérisé en ce que**
l'agent de réticulation est choisi parmi un groupe comprenant le glyoxal, le 2,2'-bioxirane, la polyéthylèneglycoldiamine, la divinylsulfone et l'éther diglycidylique de 1,4-butanediol.

7. Implant ophtalmologique (1) selon l'une quelconque des revendications 1 à 6,
**caractérisé en ce que**
le polymère hydrophile comprend de l'acide alginique et/ou un alginate et est complexé par des ions métalliques, notamment par des ions calcium.

8. Implant ophtalmologique (1) selon l'une quelconque des revendications 1 à 7,
**caractérisé en ce que**
l'au moins un inhibiteur d'angiogenèse (16) est immobilisé sur le corps de base (2).

9. Implant ophtalmologique (1) selon la revendication 8,
**caractérisé en ce que**
l'au moins un inhibiteur d'angiogenèse (16) est lié de manière covalente à l'au moins un polymère hydrophile et/ou **en ce que** l'au moins un polymère hydrophile et l'au moins un inhibiteur d'angiogenèse (16) sont présents sous forme de réseau interpénétrant.

10. Implant ophtalmologique (1) selon l'une quelconque des revendications 1 à 9,
**caractérisé en ce que**
l'au moins une feuille de support (12) est constituée au moins principalement d'un polyamide et/ou d'un polyimide et/ou d'un polylactide-co-glycolide.

11. Outil d'implantation (8) comprenant une chambre de chargement (7) dans laquelle au moins un implant ophtalmologique (1) selon l'une quelconque des revendications 1 à 10 est agencé pour être implanté sur une rétine (6) d'un œil (4) d'un patient humain ou animal.

12. Outil d'implantation (8) selon la revendication 11, **caractérisé en ce que**
celui-ci comporte une pointe d'injecteur (9) d'une longueur d'au moins 18 mm et/ou d'au plus 30 mm et/ou une pointe d'injecteur (9) d'un diamètre intérieur d'au moins 0,8 mm et/ou d'au plus 2,5 mm.
